# EUROPEAN PATENT SPECIFICATION

(11) **EP 2 690 394 B1**
(45) Date of publication and mention of the grant of the patent: **05.05.2021**
(21) Application number: 13178046.2
(22) Date of filing: 25.07.2013
(51) Int. Cl.: H04N 13/246

(54) **Yarn monitoring device and yarn winding unit**
Garnüberwachungsvorrichtung und Garnwickeleinheit
Dispositif de surveillance de fil et machine de bobinage de fil

(30) Priority: 27.07.2012 JP 2012167240
(43) Date of publication of application: 29.01.2014
(73) Proprietor: Murata Machinery, Ltd., Kyoto-shi, Kyoto 601-8326 (JP)
(72) Inventor: Minamino, Katsushi, Kyoto 612-8686 (JP); Nakade, Kazuhiko, Kyoto 612-8686 (JP)
(74) Representative: Stöckeler, Ferdinand

(56) References cited:
- EP-A2- 2 426 482
- WO-A1-2012/051730
- DE-A1- 4 028 465
- US-A- 5 054 317
- VITOR CARVALHO ET AL: "Yarn parameterization correlation using optical and capacitive sensors approaches", IECON 2009 - 35TH ANNUAL CONFERENCE OF IEEE INDUSTRIAL ELECTRONICS (IECON 2009) - 3-5 NOV. 2009 - PORTO, PORTUGAL, IEEE, PISCATAWAY, NJ, USA, 3 November 2009 (2009-11-03), pages 1941-1946, XP031629504, ISBN: 978-1-4244-4648-3

## Description

### BACKGROUND OF THE INVENTION

### 1. Field of the Invention

The present invention relates to a yarn monitoring device and a yarn winding unit that are adapted to, for example, a spinning machine or an automatic winder.

### 2. Description of the Related Art

In Japanese Patent Application Laid-open No. 2009-190841, a yarn quality measuring device is described as a yarn monitoring device. This yarn quality measuring device includes two yarn irregularity sensors arranged along a yarn path of a running yarn. The yarn quality measuring device calculates a speed of the running yarn by performing pattern matching of waveforms obtained by the two yarn irregularity sensors, and changes a sampling frequency of thickness irregularity detection in accordance with the calculated yarn speed.

In the yarn winding unit that is adapted to, for example, a spinning machine or an automatic winder or such-like, it is extremely important to correctly assess the quality of the running yarn in order to maintain the quality of the wound yarn. The quality of the yarn can be assessed by checking for presence or absence of yarn defects, faulty yarn joints, and/or a twisted state of the yarn.

US 5 054 317 A discloses a device for monitoring parameters of a running thread-like test material, which comprises an optical measuring element and a capacitive measuring element arranged one behind the other. A sum or a difference of the signals from the measuring elements is evaluated.

WO 2012/051730 A1 discloses a yarn clearer comprising a measurement head. The measurement head comprises a capacitive measuring cell and an optical measuring cell. Detected yarn characteristics are compared with predetermined quality criteria.

VITOR CARVALHO ET AL: "Yarn parameterization correlation using optical and capacitive sensors approaches", IECON 2009 - 35TH ANNUAL CONFERENCE OF IEEE INDUSTRIAL ELECTRONICS (IECON 2009) - 3-5 NOV. 2009 - PORTO, PORTUGAL, IEEE, PISCATAWAY, NJ, USA, 3 November 2009 (2009-11-03), pages 1941-1946, disclose usage of optical and capacitive sensor approaches for yarn parameterization.

EP 2 426 482 A2 discloses a textile material monitoring device and a yarn winding apparatus comprising such a textile material monitoring device.

DE 40 28 465 A1 discloses a yarn joint monitoring device for assessing the quality of a yarn joint. The joining device, a measuring unit and a cutter are activated once the system senses a rupture in the yarn.

### SUMMARY OF THE INVENTION

It is an object of the present invention to provide a yarn quality assessing method employing a yarn monitoring device and a yarn winding unit that are capable of correctly assessing a yarn quality of a running yarn.

This object is achieved by a method for assessing the quality of a running yarn according to claim 1.

A yarn monitoring device used in the present invention includes a first sensor adapted to detect a thickness of a running yarn and to obtain a thickness detection value; a second sensor adapted to detect a fiber volume of the running yarn and to obtain a fiber volume detection value; and a yarn quality assessing section adapted to assess yarn quality of

the running yarn based on the thickness detection value detected by the first sensor and the fiber volume detection value detected by the second sensor.

A yarn winding unit used in the invention includes the above yarn monitoring device; a yarn supplying device that is arranged upstream of the yarn monitoring device along a yarn path of the running yarn and that is adapted to supply the yarn; and a winding device that is arranged downstream of the yarn monitoring device along the yarn path and that is adapted to wind the yarn.

### BRIEF DESCRIPTION OF THE DRAWINGS

FIG. 1 is a perspective view of a spinning unit that serves as a yarn winding unit in the present invention;
FIG. 2 is a block diagram of a yarn monitoring device of the spinning unit shown in FIG. 1;
FIGS. 3A and 3B are charts for explaining a distance correction process performed by the yarn monitoring device shown in FIG. 2;
FIG. 4 is a chart for explaining a yarn defect assessing process performed by the yarn monitoring device shown in FIG. 2;
FIG. 5 is a chart for explaining a faulty yarn joint assessing process performed by the yarn monitoring device shown in FIG. 2; and
FIG. 6 is a chart for showing the states of a yarn in terms of a thickness and a fiber volume of the yarn.

### DETAILED DESCRIPTION

Exemplary embodiments of the present invention are explained in detail below with reference to the accompanying drawings. In the drawings, the parts that are identical or equivalent have been assigned the same reference numerals, and the description thereof is not repeated.

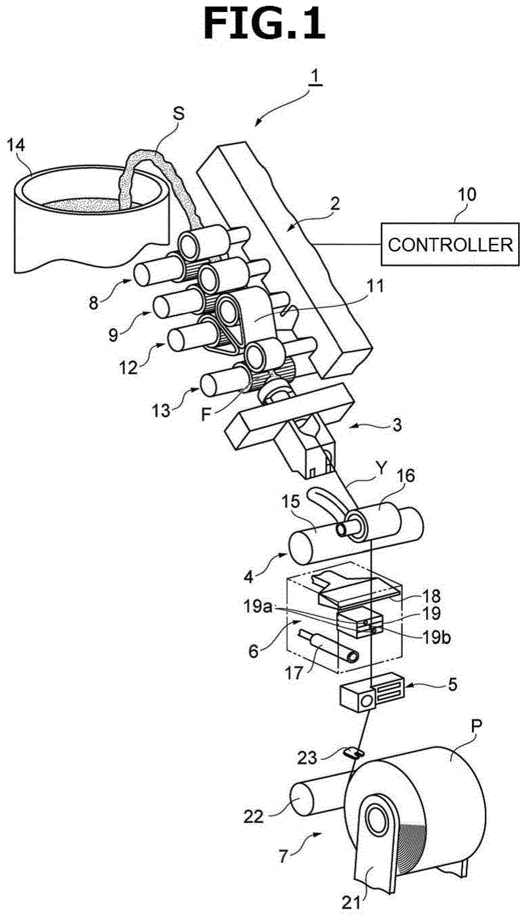

As shown in FIG. 1, a spinning unit (yarn winding unit) 1 forms a yarn Y and winds the yarn Y to form a package P. In the spinning unit 1, a drafting device (yarn supplying device) 2, a spinning device (yarn supplying device) 3, a yarn delivering device 4, a yarn joining device 6, a yarn monitoring device 5, and a winding device 7 are arranged in the mentioned order from an upstream side along a running path of the yarn Y. That is, the drafting device 2 and the spinning device 3 are arranged upstream with respect to the yarn monitoring device 5 along the path of the yarn Y (hereinafter, "yarn path"), whereas the winding device 7 is arranged downstream with the yarn monitoring device 5 along the yarn path. The yarn joining device 6 is arranged between the yarn supplying device (the drafting device 2 and the spinning device 3) and the yarn monitoring device 5 along the yarn path. The spinning unit 1 also includes a controller 10 that performs an overall control of the spinning unit 1.

A spinning machine is formed by arranging multiple spinning units 1 side by side. The yarn joining device 6 is movable between the spinning units 1 and performs a yarn joining operation in a spinning unit 1 in which the yarn Y has been cut. That is, the yarn joining device 6 is shared among a plurality of the spinning units 1. However, a separate yarn joining device 6 can be provided for each of the spinning units 1. The yarn Y can be cut when the yarn Y runs out due to some reason or when a yarn defect needs to be removed.

The drafting device 2 includes a pair of back rollers 8, a pair of third rollers 9, a pair of middle rollers 12 with an apron belt 11 stretched around each of the middle rollers 12, and a pair of front rollers 13. The drafting device 2 drafts a sliver S from a can 14 with each pair of rollers 8, 9, 12, and 13 to form a fiber bundle F.

The spinning device 3 is an air spinning device that twists the fiber bundle F formed by the drafting device 2 by subjecting the fiber bundle F to a swirling airflow to form the yarn Y. More specifically, the spinning device 3 includes a spinning chamber, a fiber guiding member, a swirling airflow producing nozzle, and a hollow guide shaft (not shown). The fiber guiding member guides the fiber bundle F formed by the drafting device 2 into the spinning chamber. The swirling airflow producing nozzle produces a swirling airflow inside the spinning chamber, causing the fiber ends of the fiber bundle F introduced into the spinning chamber to turn and swirl. The hollow guide shaft guides the spun yarn Y from the spinning chamber to outside of the spinning device 3.

The yarn delivering device 4 includes a delivery roller 15 and a nip roller 16. The yarn delivering device 4 nips the yarn Y formed by the spinning device 3 between the two rollers 15 and 16 and delivers it to the winding device 7.

The yarn monitoring device 5 assesses a yarn quality of the running yarn Y. The yarn monitoring device 5 has a function of cutting the yarn Y when a yarn defect is found in the running yarn Y. The function of cutting of the yarn Y can be realized by a structure that is separate from the yarn monitoring device 5, for example, by independently arranging a cutter or by stopping the swirling airflow in the spinning device 3.

The yarn joining device 6 includes a suction pipe 17, a suction mouth 18, and a splicer 19. The suction pipe 17 is pivotably supported. The suction pipe 17 catches a yarn end of the yarn Y spun by the spinning device 3 and guides it to the splicer 19. The suction mouth 18 is also pivotably supported. The suction mouth 18 catches a yarn end of the yarn Y from the package P and guides it to the splicer 19. The splicer 19 joins the two yarn ends guided by the suction pipe 17 and the suction mouth 18. The yarn Y from the package P includes a yarn defect. When the suction mouth 18 sucks and catches the yarn Y, the yarn defect gets sucked into the suction mouth 18 and removed. The splicer 19 thereby joins the yarn-defect removed yarn Y from the package P and the yarn Y spun by the spinning device 3.

The splicer 19 uses the swirling airflow to form a yarn joint. The splicer 19 includes an untwisting section 19a and a twisting section 19b. The untwisting section 19a untwists the yarn end of the yarn Y from the package P (one yarn end) and the yarn end of the yarn Y spun by the spinning device 3 (the other yarn end) after the cutting of the yarn Y. The twisting section 19b twists the two untwisted yarn ends by the swirling airflow. In place of this splicer 19, a disc-type splicer can be employed.

The winding device 7 winds the yarn Y to form the package P. The winding device 7 includes a cradle arm 21, a winding drum 22, and a traverse device 23. The cradle arm 21 rotatably supports the package P, and brings an outer surface of the package P into contact with an outer surface of the winding drum 22 with an appropriate pressure. The winding drum 22 causes the package P, which is brought into contact with the winding drum 22 by the cradle arm 21, to rotate. The traverse device 23 traverses the yarn Y within a given width over the package P rotated by the winding drum 22. The spinning unit 1 can wind the yarn Y into not only the cylindrical package P as shown in FIG. 1 but also a cone-shaped package P. The shape of the package P wound by the spinning unit 1 is not particularly limited.

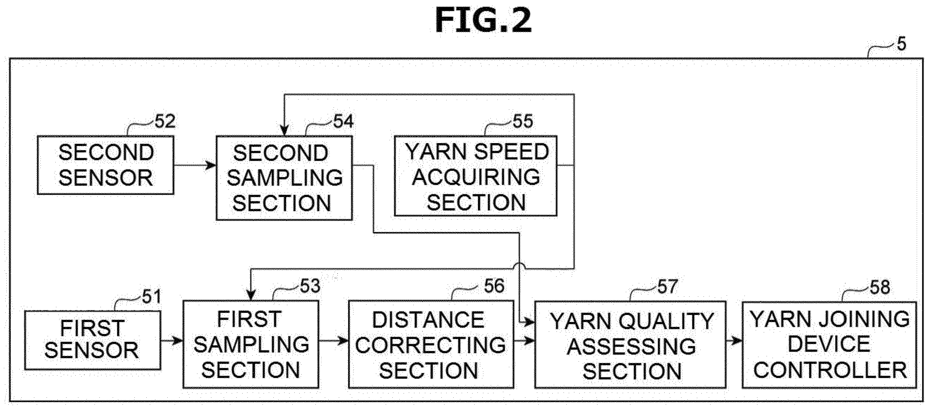

The yarn monitoring device 5 is explained below in more detail. As shown in FIG. 2, the yarn monitoring device 5 includes a first sensor 51 and a second sensor 52. The first sensor 51 and the second sensor 52 are arranged along the yarn path such that the first sensor 51 is located upstream of the second sensor 52. Detection positions of the first sensor 51 and the second sensor 52 are separated from each other by a given distance along the yarn path.

The first sensor 51 is an optical sensor that includes a light emitter and a light receiver arranged one on either side of the yarn path. The first sensor 51 detects a thickness of the running yarn Y based on an amount of light received by the light receiver through the yarn Y after the light is emitted by the light emitter. The thickness of the yarn Y corresponds to a surface area or a width per given length of the yarn Y that is projected on a given surface parallel to the yarn path.

The second sensor 52 is a capacitance sensor that includes a pair of electrodes arranged one on either side of the yarn path. The second sensor 52 detects a fiber volume of the running yarn Y based on the electrostatic capacitance between the pair of electrodes with the yarn Y running between them. The fiber volume of the yarn Y corresponds to a mass of the yarn Y per given length.

The yarn monitoring device 5 further includes a first sampling section 53, a second sampling section 54, and a yarn speed acquiring section 55. The yarn speed acquiring section 55 acquires a yarn speed (that is, a running speed of the yarn Y), and outputs a yarn speed signal (a signal indicating the yarn speed) to the first sampling section 53 and the second sampling section 54. The yarn speed acquiring section 55 acquires the yarn speed detected by a separately arranged yarn speed sensor, or calculates the approximate yarn speed based on the number of rotations of the winding drum 22 per unit time in the winding device 7, or calculates the yarn speed based on a formation speed of the yarn Y by the spinning device 3.

The yarn speed acquiring section 55 can also acquire the yarn speed in the following manner. The first sensor 51 and another similar optical sensor can be arranged along the yarn path. Pattern matching of the waveforms of the two optical sensors is performed so that the yarn speed can be calculated based on a distance between the detection positions of the two optical sensors and a time lag of the waveforms of the two optical sensors. Alternatively, the yarn speed can be calculated by the above process by using the second sensor 52 and another similar capacitance sensor that are arranged along the yarn path.

The first sampling section 53 adjusts a sampling time (a given time point) based on the yarn speed signal received from the yarn speed acquiring section 55, and samples a thickness detection value (a signal indicating the thickness) from the first sensor 51 at that sampling time. The first sampling section 53 adjusts the sampling time so that the thickness signal can be sampled for every given length of the yarn Y.

The second sampling section 54 adjusts a sampling time (a given time point) based on the yarn speed signal received from the yarn speed acquiring section 55, and samples a fiber volume detection value (a signal indicating the fiber volume) from the second sensor 52 at that sampling time. The second sampling section 54 adjusts the sampling time so that the fiber volume signal can be sampled for every given length of the yarn Y.

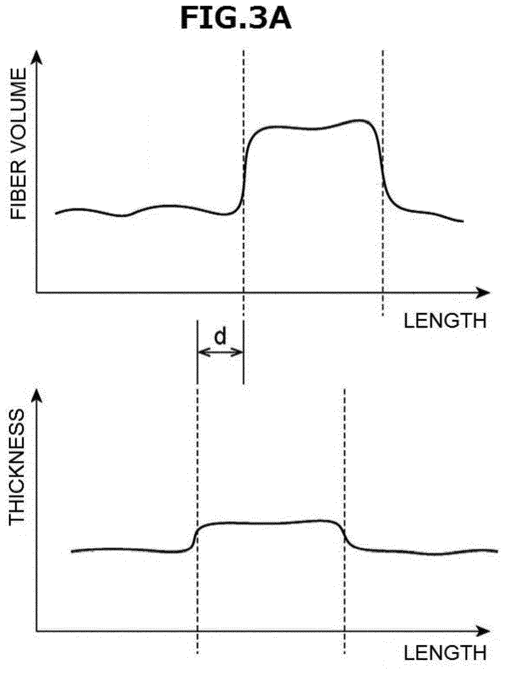

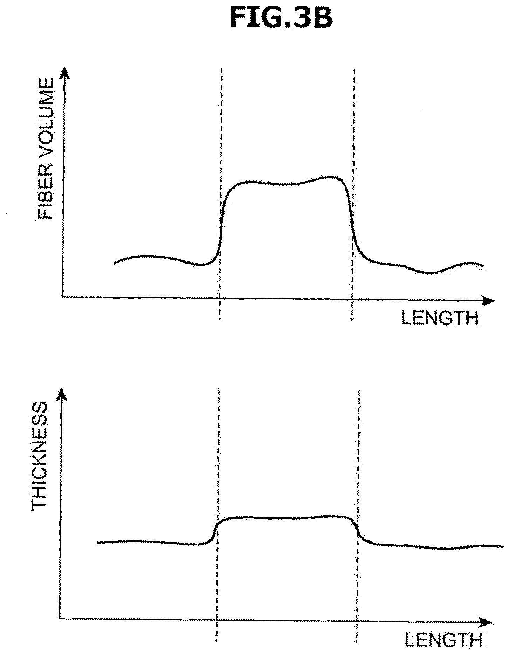

The yarn monitoring device 5 further includes a distance correcting section (adjusting section) 56, a yarn quality assessing section 57, and a yarn joining device controller 58. Because the first sensor 51 is located upstream of the second sensor 52 along the yarn path, as explained above, if attention is focused on a certain portion of the running yarn Y, as shown in FIG. 3A, the detection position of the thickness of that portion by the first sensor 51 precedes the detection position of the fiber volume of that portion by the second sensor 52 by a given distance d. Therefore, as shown in FIG. 3B, the distance correcting section 56 delays the thickness detection value received from the first sampling section 53 by the given distance d so that the thickness detection value and the fiber volume detection value of the same portion can be brought into correspondence and compared with each other.

The yarn quality assessing section 57 assesses the yarn quality of the running yarn Y based on the thickness detection value received from the distance correcting section 56 and the fiber volume detection value received from the second sampling section 54. That is, the yarn quality assessing section 57 assesses the yarn quality of the running yarn Y based on the thickness detection value detected by the first sensor 51 and the fiber volume detection value detected by the second sensor 52. The yarn quality assessing section 57 assesses the yarn quality in terms of presence or absence of a yarn defect and of a faulty yarn joint.

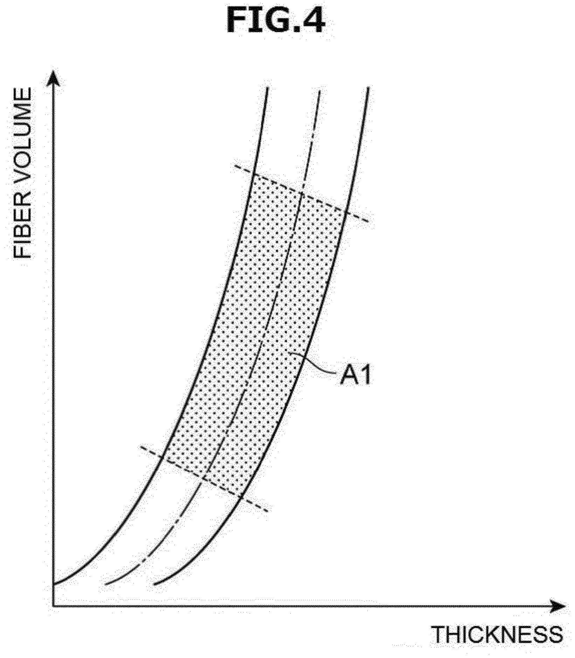

An assessment process of the presence or absence of a yarn defect performed by the yarn quality assessing section 57 is explained below. As shown in FIG. 4, the yarn quality assessing section 57 stores therein an allowable yarn defect range A1 that is established by using at least the thickness and the fiber volume of the yarn Y as parameters. The allowable yarn defect range A1 is defined as follows. Assuming a fiber density to be constant if the yarn Y is appropriately twisted, the fiber volume of the yarn Y is proportional to π×(thickness of the yarn Y/2)². A dashed-dotted line shown in FIG. 4 represents the relationship between the thickness and the fiber volume. The allowable yarn defect range A1 is defined around this relationship between the thickness and the fiber volume by setting an allowable range of fiber density (represented by solid lines on two sides of the dashed line in FIG. 4) and also setting upper and lower limits (represented by broken lines in FIG. 4). The allowable yarn defect range A1 can be defined in accordance with the type of the yarn defect, such as a nep, a slub, and a lengthy defective portion.

The yarn quality assessing section 57 determines whether the thickness detection value and the fiber volume detection value of a given portion of the yarn Y whose integrated value (integral) of the thickness detection values of the yarn Y of a given length exceeds a given integrated value are within the allowable yarn defect range A1. If the thickness detection value and the fiber volume detection value of the given portion are within the allowable yarn defect range A1, the yarn quality assessing section 57 assesses the given portion not to have a yarn defect. If the thickness detection value and the fiber volume detection value of the given portion are not within the allowable yarn defect range A1 (that is, if the thickness detection value and the fiber volume detection value are outside the allowable yarn defect range A1), the yarn quality assessing section 57 judges that the given portion has a yarn defect.

If the yarn quality assessing section 57 determines that the given portion has a yarn defect, it causes the yarn monitoring device 5 to cut the yarn Y and also causes the yarn joining device controller 58 to control the yarn joining device 6 to perform a yarn joining operation. In this manner, the portion that is judged as having a yarn defect is removed from the yarn Y.

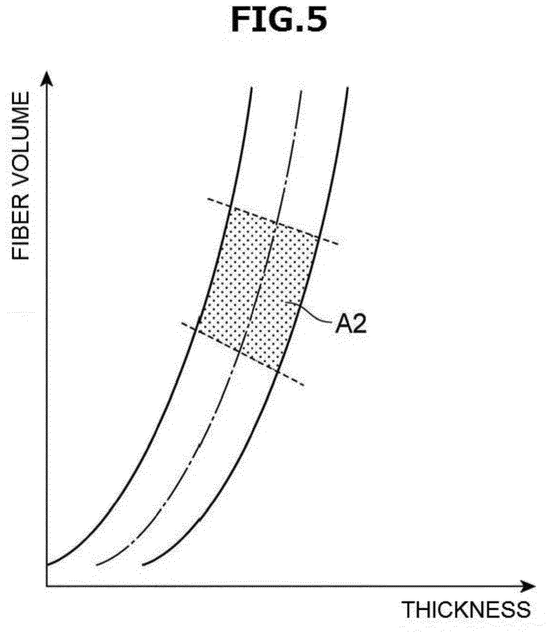

An assessment process of the presence or absence of a faulty yarn joint performed by the yarn quality assessing section 57 is explained below. As shown in FIG. 5, the yarn quality assessing section 57 stores therein an allowable faulty yarn joint range A2 that is established by using at least the thickness and the fiber volume of the yarn Y as parameters. The allowable faulty yarn joint range A2 is defined as follows. Assuming the fiber density to be constant if the yarn Y is appropriately twisted, similar to FIG. 4, a dashed-dotted line shown in FIG. 5 represents the relationship between the thickness and the fiber volume. The allowable faulty yarn joint range A2 is defined around this relationship between the thickness and the fiber volume by setting an allowable range of fiber density (represented by solid lines on two sides of the dashed line in FIG. 5) and also setting upper and lower limits (represented by broken lines in FIG. 5).

The yarn quality assessing section 57 determines whether the thickness detection value and the fiber volume detection value of a yarn joint of the yarn Y whose integrated value (integral) of the fiber volume detection values of the yarn Y of a given length exceeds a given integrated value are inside the allowable faulty yarn joint range A2. If the thickness detection value and the fiber volume detection value of the yarn joint are within the allowable faulty yarn joint range A2, the yarn quality assessing section 57 judges the yarn joint as not faulty. If the thickness detection value and the fiber volume detection value of the yarn joint are not within the allowable faulty yarn joint range A2 (that is, if the thickness detection value and the fiber volume detection value are outside the allowable faulty yarn joint range A2), the yarn quality assessing section 57 judges the yarn joint as faulty. A yarn joint of the running yarn Y is a portion of the running yarn Y which has been cut once and then joined by the yarn joining device 6.

If the yarn quality assessing section 57 judges the yarn joint as faulty, it causes the yarn monitoring device 5 to cut the yarn Y and sends a command signal to the controller 10 via the yarn joining device controller 58. Upon receiving the command signal, the controller 10 causes the yarn joining device 6 to perform the yarn joining operation. In this manner, the yarn joint that is judged as faulty is removed from the yarn Y. Furthermore, when a yarn joint is judged as faulty, the yarn quality assessing section 57 sends a command signal to the controller 10 via the yarn joining device controller 58 to prevent faulty yarn joints from occurring subsequently. Upon receiving the command signal, the controller 10 controls the yarn joining operation performed by the yarn joining device 6 by controlling at least one of the untwisting section 19a and the twisting section 19b. The controller 10, for example, adjusts at least one of an untwisting duration (an operation period of the untwisting section 19a) of the yarn Y and a twisting duration (an operation period of the twisting section 19b) of the yarn Y in the yarn joining device 6. Alternatively, the controller 10 can also adjust a joint length of the yarn Y in the yarn joining device 6. The controller 10 can also give an operator an alert message pertaining to a faulty yarn joint on, for example, a display section, thereby urging the operator to adjust at least one of a pressure of air supplied to the untwisting section 19a, a pressure of air supplied to the twisting section 19b, a jet angle of air blown from the untwisting section 19a, and a jet angle of air blown from the twisting section 19b.

As explained above, the yarn monitoring device 5 can detect the thickness and the fiber volume of the running yarn Y and thereby acquire information pertaining to the fiber density, which is otherwise difficult to assess based only on either one of the thickness detection value and the fiber volume detection value. Therefore, the yarn quality of the running yarn Y can be correctly assessed by the yarn monitoring device 5 and the spinning unit 1 incorporating the yarn monitoring device 5. Particularly, because the information pertaining to the fiber density can be acquired, the quality of the yarn Y in terms of the fiber density can be correctly assessed.

In the yarn monitoring device 5, the distance correcting section 56 adjusts the thickness detection value received from the first sampling section 53 so that the thickness detection value and the fiber volume detection value of a given portion of the running yarn Y are correlated. The yarn quality assessing section 57 assesses the yarn quality of the yarn Y in the given portion based on the thickness detection value and the fiber volume detection value of that very portion. Consequently, even though the first sensor 51 and the second sensor 52 are located at different positions along the yarn path, the yarn quality of a given portion of the running yarn Y can be correctly assessed based on the thickness detection value and the fiber volume detection value of the same portion of the running yarn Y.

In the yarn monitoring device 5, the first sampling section 53 samples the thickness detection value detected by the first sensor 51, and the second sampling section 54 samples the fiber volume detection value detected by the second sensor 52, based on the running speed of the yarn Y obtained by the yarn speed acquiring section 55. The distance correcting section 56 adjusts the thickness detection value received from the first sampling section 53 based on the sampled thickness detection value and the sampled fiber volume detection value. Consequently, because the thickness detection value and the fiber volume detection value can be sampled for every given length (for example, per millimeter) of the yarn Y regardless of the running speed of the yarn Y, it is easier to perform the adjustment required for correlating the thickness detection value and the fiber volume detection value for the same portion of the running yarn Y.

In the yarn monitoring device 5, the yarn quality assessing section 57 assesses the yarn quality in terms of the presence or absence of a yarn defect. Consequently, yarn defects can be removed by the yarn joining device 6 in the spinning unit 1.

In the yarn monitoring device 5, if the thickness detection value and the fiber volume detection value of the same portion of the running yarn Y are outside the allowable yarn defect range A1, which is established by using at least the thickness and the fiber volume as parameters, the yarn quality assessing section 57 judges that the portion has a yarn defect. Consequently, whether a given portion has a yarn defect can be correctly judged based on the information pertaining to the fiber density, which is otherwise difficult to assess based only on either one of the thickness detection value and the fiber volume detection value.

In the yarn monitoring device 5, the yarn quality assessing section 57 assesses the yarn quality in terms of the presence or absence of a faulty yarn joint. Consequently, the spinning unit 1, based on this assessment result, can remove the faulty yarn joint by the yarn joining device 6, and adjust the operation of the yarn joining device 6 (untwisting duration, twisting duration, and/or air pressure) so that faulty yarn joints can be reduced.

In the yarn monitoring device 5, if the thickness detection value and the fiber volume detection value of a yarn joint of the running yarn Y are outside the allowable faulty yarn joint range, which is established by using at least the thickness and the fiber volume as parameters, the yarn quality assessing section 57 judges the yarn joint as faulty. Consequently, whether a given yarn joint is faulty can be correctly assessed based on the information pertaining to the fiber density, which is otherwise difficult to assess based only on either one of the thickness detection value and the fiber volume detection value.

In the yarn monitoring device 5, because the first sensor 51 is an optical sensor, the thickness of the running yarn Y can be detected accurately.

In the yarn monitoring device 5, because the second sensor 52 is a capacitance sensor, the fiber volume of the running yarn Y can be detected accurately.

In the spinning unit 1, when the yarn quality assessing section 57 of the yarn monitoring device 5 assesses the yarn quality in terms of the presence or absence of a faulty yarn joint, the controller 10 controls at least one of the untwisting section 19a and the twisting section 19b, thereby controlling the yarn joining operation performed by the yarn joining device 6. Consequently, faulty yarn joints made by the yarn joining device 6 can be reliably reduced.

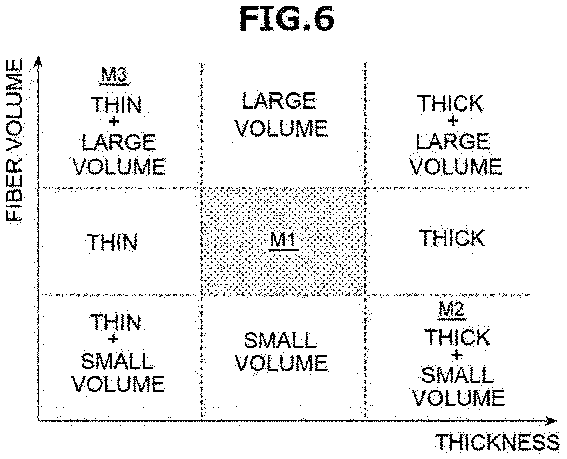

An embodiment of the present invention is explained above; however, the present invention is not limited thereto. Besides the presence or absence of a yarn defect, and the presence or absence of a faulty yarn joint, the yarn monitoring device 5 according to the present embodiment can also assess the yarn quality in terms of the twisted state of the yarn Y. FIG. 6 is a chart showing the states of the yarn Y in terms of the thickness and the fiber volume. As shown in FIG. 6, a range M1 represents an appropriate thickness and fiber volume of the yarn Y. If a given portion of the yarn Y falls in a range M2, in which the yarn Y is thicker but has a smaller fiber volume with respect to the appropriate thickness and appropriate fiber volume of the range M1, this indicates that the given portion of the yarn Y has lost more than an appropriate volume of fiber during the untwisting operation (excessive untwisting) and has been inadequately twisted during the twisting operation. On the other hand, if a given portion of the yarn Y falls in a range M3, in which the yarn Y is thinner and has a larger fiber volume with respect to the appropriate thickness and appropriate fiber volume of the range M1, this indicates that the given portion of the yarn Y has lost less than an appropriate volume of fiber during the untwisting operation (inadequate untwisting) and has been excessively twisted during the twisting operation. In a given portion of the yarn Y, when the thickness is the same as that of the range M1 but the fiber volume is larger than that of the range M1 or when both the thickness and the fiber volume of the given portion are larger than the appropriate thickness and fiber volume of the range M1, it indicates that less than an appropriate volume of fiber has been lost during the untwisting operation, or that the yarn joint has been made with at least one yarn end doubled over itself. When both the thickness and the fiber volume of the given portion of the yarn Y are smaller than the appropriate thickness and fiber volume of the range M1 or when the thickness is the same as that of the range M1 but the fiber volume is smaller than that of the range M1, it indicates that more than an appropriate volume of fiber has been lost during the untwisting operation. In the given portion of the yarn Y, when the fiber volume is the same as that of the range M1 but the given portion is thinner than that of the range M1, it indicates that the yarn Y in the given portion has been excessively twisted during the twisting operation. Conversely, when the fiber volume is the same as that of the range M1 but the given portion of the yarn Y is thicker than that of the range M1 in a given portion, it indicates that the yarn Y in the given portion has been inadequately twisted.

The first sensor 51 according to the present invention need not be limited to an optical sensor, i.e., any sensor that can detect the thickness of the running yarn Y can be used as the first sensor 51. The second sensor 52 according to the present invention need not be limited to a capacitance sensor, i.e., any sensor that can detect the fiber volume of the running yarn Y can be used as the second sensor 52. As for the positional relation between the first sensor 51 and the second sensor 52 according to the present invention, the first sensor 51 can be located upstream of the second sensor 52 along the yarn path, or the second sensor 52 can be located upstream of the first sensor 51 along the yarn path. The correcting section arranged in the yarn monitoring device 5 according to the present invention need not be limited to the distance correcting section 56; the yarn monitoring device 5 can include any other correcting section that can adjust at least one of the thickness detection value detected by the first sensor 51 and the fiber volume detection value detected by the second sensor 52 so that the thickness and the fiber volume of the same portion in the running yarn Y are correlated even though the first sensor 51 and the second sensor 52 are located at different positions along the yarn path of the running yarn Y. The sampling sections according to the present invention need not be limited to the first sampling section 53 and the second sampling section 54. For example, the yarn monitoring device 5 can include a single sampling section that can sample the thickness detection value detected by the first sensor 51 and the fiber volume detection value detected by the second sensor 52 based on the running speed acquired by the yarn speed acquiring section 55.

The yarn winding unit used in the present invention need not be limited to the spinning units 1 that constitute the spinning machine but can be other yarn winding units, such as winder units that constitute an automatic winder. In the spinning unit 1 explained above, the yarn supplying device includes the drafting device 2 and the spinning device 3; however, any yarn supplying device that, for example, can supply the yarn Y from a bobbin around which the yarn Y is wound can be used as the yarn supplying device. If the yarn winding unit according to the present invention is a spinning unit, the spinning unit need not draw the yarn Y from the spinning machine by the delivery roller 15 and the nip roller 16; the yarn Y can be pooled by a yarn pooling roller provided downstream of the spinning device 3 and drawn from the spinning device 3 by the yarn pooling roller.

In the above embodiment, a separate yarn joining device 6 can be provided for each spinning unit 1, or a movable yarn joining device 6 can be shared among multiple spinning units 1. Likewise, a separate controller 10 can be provided for each spinning unit 1, or a controller 10 can be shared among the spinning units 1. The yarn monitoring device 5 can be provided downstream of the spinning device 3 and upstream of the yarn joining device 6. The yarn quality in terms of the presence or absence of the yarn defect can be correctly assessed with this arrangement.

A yarn monitoring device used in the present invention includes a first sensor that detects a thickness of a running yarn and obtains a thickness detection value; a second sensor that detects a fiber volume of the running yarn and obtains a fiber volume detection value; and a yarn quality assessing section that assesses yarn quality of the running yarn based on the thickness detection value detected by the first sensor and the fiber volume detection value detected by the second sensor.

In the yarn monitoring device used in the present embodiment, the thickness and the fiber volume of the running yarn are detected so that information pertaining to a fiber density, which is otherwise difficult to assess based only on one of the thickness detection value and the fiber volume detection value, can be acquired. Consequently, the yarn quality of the running yarn can be correctly assessed by the yarn monitoring device. The thickness of the yarn corresponds to a surface area or a width per given length of the yarn projected on a given surface that is parallel to a yarn path. The fiber volume of the yarn corresponds to a mass of the yarn per given length of the yarn.

According to another aspect of the invention, it is preferable that the yarn quality pertains to a fiber density of the yarn. As mentioned above, because the information pertaining to the fiber density can be acquired, the yarn quality in terms of the fiber density can be correctly assessed.

The yarn monitoring device used in the invention preferably further includes an adjusting section that adjusts at least one of the thickness detection value and the fiber volume detection value so that the thickness and the fiber volume of a given portion in the running yarn are correlated even if the first sensor and the second sensor are located at different positions along a yarn path of the running yarn, wherein the yarn quality assessing section assesses the yarn quality of the given portion based on the thickness detection value and the fiber volume detection value of the given portion adjusted by the adjusting section. With this structure, even though the first sensor and the second sensor are located at different positions along the yarn path, the yarn quality of a given portion of the running yarn can be correctly assessed based on the thickness detection value and the fiber volume detection value of that portion of the running yarn.

The yarn monitoring device used in the invention preferably further includes a yarn speed acquiring section that acquires a running speed of the yarn; and a sampling section that samples the thickness detection value and the fiber volume detection value based on the running speed acquired by the yarn speed acquiring section, wherein the adjusting section adjusts at least one of the thickness detection value and the fiber volume detection value based on the thickness detection value and the fiber volume detection value sampled by the sampling section. With this structure, because the thickness detection value and the fiber volume detection value can be sampled for every given length of the yarn regardless of the running speed of the yarn, the adjustment required for correlating the thickness detection value and the fiber volume detection value for the same portion of the running yarn can be facilitated.

The yarn quality assessing section assesses the yarn quality in terms of presence or absence of a yarn defect. With this structure, by incorporating the yarn monitoring device into a yarn winding unit that includes a yarn joining device, yarn defects can be removed by using the yarn joining device.

The yarn quality assessing section judges a portion as having a yarn defect if the thickness detection value and the fiber volume detection value of the given portion in the running yarn are outside an allowable yarn defect range that is established by using at least the thickness and the fiber volume as parameters. With this structure, whether a given portion has a yarn defect can be correctly assessed based on the information pertaining to the fiber density, which is otherwise difficult to assess based only on one of the thickness detection value and the fiber volume detection value of the yarn.

The yarn quality assessing section assesses the yarn quality in terms of presence or absence of a faulty yarn joint. With this structure, by incorporating the yarn monitoring device into the yarn winding unit that includes the yarn joining device, faulty yarn joints can be removed by using the yarn joining device, and the operation of the yarn joining device can be adjusted so that faulty yarn joints do not occur subsequently.

The yarn quality assessing section judges a yarn joint as faulty if the thickness detection value and the fiber volume detection value of the yarn joint in the running yarn are outside an allowable faulty yarn joint range that is established by using at least the thickness and the fiber volume as parameters. With this structure, whether a given yarn joint is faulty can be correctly assessed based on the information pertaining to the fiber density, which is otherwise difficult to assess based only on one of the thickness detection value and the fiber volume detection value of the yarn. A yarn joint of the running yarn is a portion of the running yarn which has been cut once and then joined.

The first sensor is preferably an optical sensor. With this structure, the thickness of the running yarn can be detected accurately.

The second sensor is preferably a capacitance sensor. With this structure, the fiber volume of the running yarn can be detected accurately.

A yarn winding unit used in the invention includes the above yarn monitoring device; a yarn supplying device that is arranged upstream of the yarn monitoring device along a yarn path of the running yarn and that supplies the yarn; and a winding device that is arranged downstream of the yarn monitoring device along the yarn path and that winds the yarn.

The yarn winding unit preferably includes the above-mentioned yarn monitoring device. Consequently, the yarn quality of the running yarn can be correctly assessed.

The yarn winding unit preferably further includes a yarn joining device that is arranged between the yarn supplying device and the yarn monitoring device along the yarn path and that joins the yarn that has been cut. With this structure, because the yarn monitoring device correctly assesses the presence or absence of yarn defects, the yarn can be joined after the removal of the yarn defects, and the yarn can be wound again.

The yarn winding unit preferably further includes a controller that controls the yarn joining device when the yarn quality assessing section of the yarn monitoring device assesses the yarn quality in terms of a faulty yarn joint. With this structure, occurrence of faulty joints made by the yarn joining device can be restrained.

The yarn joining device preferably includes an untwisting section that untwists two yarn ends of the yarn that has been cut, and a twisting section that twists the two yarn ends that have been untwisted by the untwisting section, and the controller controls at least one of the untwisting section and the twisting section. With this structure, occurrence of faulty joints made by the yarn joining device can be restrained.

## Claims

1. A method for assessing quality of a running yarn using a yarn monitoring device, the yarn monitoring device comprising a first sensor (51) adapted to detect a thickness of a running yarn (Y) and to obtain a thickness detection value, a second sensor (52) adapted to detect a fiber volume of the running yarn (Y) and to obtain a fiber volume detection value, and a yarn quality assessing section (57), the method comprising the following steps performed by the yarn quality assessing section:
assessing the yarn quality in terms of presence or absence of a yarn defect by judging a portion as having a yarn defect if the thickness detection value and the fiber volume detection value of the given portion in the running yarn (Y) are outside an allowable yarn defect range (A1) that is established by using at least the thickness and the fiber volume as parameters,
if judging that the given portion has a yarn defect, causing the yarn (Y) to be cut and causing a yarn joining device controller (57) to control a yarn joining device (6) to perform a yarn joining operation, to remove the portion that is judged as having a yarn defect and to generate a yarn joint,
assessing yarn quality of the running yarn (Y) in terms of presence or absence of a faulty yarn joint based on the thickness detection value detected by the first sensor (51) and the fiber volume detection value detected by the second sensor (52), by judging the yarn joint as faulty if the thickness detection value and the fiber volume detection value of the yarn joint in the running yarn (Y) are outside an allowable faulty yarn joint range (A2) that is established by using at least the thickness and the fiber volume as parameters.

2. The method according to Claim 1, wherein the yarn quality pertains to a fiber density of the yarn.

3. The method according to any one of Claims 1 and 2, further comprising by an adjusting section (56) adjusting at least one of the thickness detection value and the fiber volume detection value so that the thickness and the fiber volume of a given portion in the running yarn (Y) are correlated even if the first sensor (51) and the second sensor (52) are located at different positions along a yarn path of the running yarn (T),
wherein the yarn quality of the given portion is assessed by the yarn quality assessing section (57) based on the thickness detection value and the fiber volume detection value of the given portion adjusted by the adjusting section (56).

4. The method according to Claim 3, further comprising:
acquiring a running speed of the yarn (Y) by a yarn speed acquiring section (55); and
by a sampling section (53, 54), sampling the thickness detection value and the fiber volume detection value based on the running speed acquired by the yarn speed acquiring section (55),
wherein at least one of the thickness detection value and the fiber volume detection value is adjusted by the adjusting section (56) based on the thickness detection value and the fiber volume detection value sampled by the sampling section (53, 54).

5. The method according to any one of Claims 1 to 4, wherein the first sensor (51) is an optical sensor.

6. The method according to any one of Claims 1 to 5, wherein the second sensor (52) is a capacitance sensor.

7. The method according to one of claims 1 to 6, wherein the allowable yarn defect range (A1) and the allowable faulty yarn joint range (A2) are respectively established around the relationship π x (T/2)² between the thickness T of the yarn and the fiber volume by setting an allowable range of fiber density and also setting upper and lower limits.

8. The method according to one of claims 1 to 7, wherein the yarn quality is assessed by the yarn quality assessing section (57) in terms of the twisted state of the yarn.

9. The method according to claim 8, wherein it is assessed by the yarn quality assessing section (57) that a given portion of the yarn (Y) has lost more than an appropriate volume of fiber during an untwisting operation and has been inadequately twisted during a twisting operation, if the given portion of the yarn (Y) falls in a range (M2), in which the yarn is thicker but has a smaller fiber volume with respect to a range (M1) of an appropriate thickness and an appropriate fiber volume.

10. The method according to claim 8 or 9, wherein it is assessed by the yarn quality assessing section (57) that a given portion of the yarn (Y) has lost less than an appropriate volume of fiber during the untwisting operation and has been excessively twisted during the twisting operation, if the given portion of the yarn (Y) falls in a range (M3), in which the yarn is thinner and has a larger fiber volume with respect to a range (M1) of an appropriate thickness and an appropriate fiber volume.

11. The method according to any one of Claims 1 to 10, further comprising supplying the yarn by a yarn supplying device (2, 3) that is arranged upstream of the yarn monitoring device (5) along a yarn path of the running yarn (y), and winding the yarn by a winding device (7) that is arranged downstream of the yarn monitoring device (5) along the yarn path.

12. The method according to Claim 11, further comprising joining the yarn (Y) that has been cut by the yarn joining device (6) that is arranged between the yarn supplying device (2, 3) and the yarn monitoring device (5) along the yarn path.

13. The method according to Claim 12, further comprising by a controller (10) controlling the yarn joining device (6) when the yarn quality assessing section (57) of the yarn monitoring device (5) assesses the yarn quality in terms of a faulty yarn joint.

14. The method according to claim 13, wherein
an untwisting section (19a) of the yarn joining device (6) untwists two yarn ends of the yarn (Y) that has been cut, and a twisting section (19b) of the yarn joining device twists the two yarn ends that have been untwisted by the untwisting section (19a), and
the controller (10) controls at least one of the untwisting section (19a) and the twisting section (19b).

## Patentansprüche

1. Ein Verfahren zum Bewerten der Qualität eines laufenden Garnes unter Verwendung einer Garnüberwachungsvorrichtung, wobei die Garnüberwachungsvorrichtung einen ersten Sensor (51), der dazu angepasst ist, eine Dicke eines laufenden Garns (Y) zu erfassen und einen Dickenerfassungswert zu erhalten, einen zweiten Sensor (52), der dazu angepasst ist, ein Faservolumen des laufenden Garns (Y) zu erfassen und einen Faservolumenerfassungswert zu erhalten, und ein Garnqualitätsbewertungssegment (57) aufweist, wobei das Verfahren die folgenden Schritte aufweist, die durch das Garnqualitätsbewertungssegment durchgeführt werden:
Bewerten der Garnqualität bezüglich eines Vorliegens oder Nichtvorliegens eines Garndefekts, indem ein Abschnitt als einen Garndefekt aufweisend beurteilt wird, falls der Dickenerfassungswert und der Faservolumenerfassungswert des gegebenen Abschnitts in dem laufenden Garn (Y) außerhalb eines zulässigen Garndefektbereichs (A1) liegen, der durch Verwendung zumindest der Dicke und des Faservolumens als Parameter festgelegt wird,
falls beurteilt wird, dass der gegebene Abschnitt einen Garndefekt aufweist, Bewirken, dass das Garn (Y) geschnitten wird, und Bewirken, dass eine Garnzusammenführungsvorrichtungssteuerung (57) eine Garnzusammenführungsvorrichtung (6) dahingehend steuert, einen Garnzusammenführungsvorgang vorzunehmen, um den Abschnitt, der als einen Garndefekt aufweisend beurteilt wird, zu beseitigen und eine Garnzusammenführung zu erzeugen,
Bewerten der Garnqualität des laufenden Garns (Y) bezüglich eines Vorliegens oder Nichtvorliegens einer fehlerhaften Garnzusammenführung auf der Basis des durch den ersten Sensor (51) erfassten Dickenerfassungswerts und des durch den zweiten Sensor (52) erfassten Faservolumenerfassungswerts, in dem die Garnzusammenführung als fehlerhaft beurteilt wird, falls der Dickenerfassungswert und der Faservolumenerfassungswert der Garnzusammenführung in dem laufenden Garn (Y) außerhalb eines zulässigen Fehlerhafte-Garnzusammenführung-Bereichs (A2) liegen, der durch Verwendung zumindest der Dicke und des Faservolumens als Parameter festgelegt wird.

2. Das Verfahren gemäß Anspruch 1, bei dem sich die Garnqualität auf eine Faserdichte des Garns bezieht.

3. Das Verfahren gemäß einem der Ansprüche 1 und 2, das ferner ein Anpassungssegment (56) aufweist, das zumindest entweder den Dickenerfassungswert und/oder den Faservolumenerfassungswert derart anpasst, dass die Dicke und das Faservolumen eines gegebenen Abschnitts in dem laufenden Garn (Y) sogar dann korreliert werden, falls sich der erste Sensor (51) und der zweite Sensor (52) an verschiedenen Positionen entlang eines Garnpfades des laufenden Garns (T) befinden,
wobei die Garnqualität des gegebenen Abschnitts durch das Garnqualitätsbewertungssegment (57) auf der Basis des Dickenerfassungswerts und des Faservolumenerfassungswerts des durch das Anpassungssegment (56) angepassten gegebenen Abschnitts bewertet wird.

4. Das Verfahren gemäß Anspruch 3, das ferner folgende Schritte aufweist:
Eruieren einer Laufgeschwindigkeit des Garns (Y) durch ein Laufgeschwindigkeitseruierungssegment (55); und
seitens eines Abtastsegments (53, 54), Abtasten des Dickenerfassungswerts und des Faservolumenerfassungswerts auf der Basis der durch das Laufgeschwindigkeitseruierungssegment (55) eruierten Laufgeschwindigkeit,
wobei zumindest entweder der Dickenerfassungswert und/oder der Faservolumenerfassungswert durch das Anpassungssegment (56) auf der Basis des Dickenerfassungswerts und des Faservolumenerfassungswerts, die durch das Abtastsegment (53, 54) abgetastet werden, angepasst wird.

5. Das Verfahren gemäß einem der Ansprüche 1 bis 4, bei dem der erste Sensor (51) ein optischer Sensor ist.

6. Das Verfahren gemäß einem der Ansprüche 1 bis 5, bei dem der zweite Sensor (52) ein Kapazitätssensor ist.

7. Das Verfahren gemäß einem der Ansprüche 1 bis 6, bei dem der zulässige Garndefektbereich (A1) und der zulässige Fehlerhafte-Garnzusammenführung-Bereich (A2) jeweils um die Beziehung π x (T/2)² zwischen der Dicke T des Garns und dem Faservolumen festgelegt werden, indem ein zulässiger Bereich einer Faserdichte eingestellt wird und auch eine Ober- und eine Untergrenze eingestellt wird.

8. Das Verfahren gemäß einem der Ansprüche 1 bis 7, bei dem die Garnqualität durch das Garnqualitätsbewertungssegment (57) bezüglich des verdrillten Zustands des Garns bewertet wird.

9. Das Verfahren gemäß Anspruch 8, bei dem durch das Garnqualitätsbewertungssegment (57) bewertet wird, dass ein gegebener Abschnitt des Garns (Y) während eines Entdrillungsvorgangs mehr als ein angemessenes Faservolumen verloren hat und während eines Verdrillungsvorgangs unzureichend verdrillt wurde, falls der gegebene Abschnitt des Garns (Y) in einen Bereich (M2) fällt, in dem das Garn bezüglich eines Bereichs (M1) einer angemessenen Dicke und eines angemessenen Faservolumens dicker ist, jedoch ein geringeres Faservolumen aufweist.

10. Das Verfahren gemäß Anspruch 8 oder 9, bei dem durch das Garnqualitätsbewertungssegment (57) bewertet wird, dass ein gegebener Abschnitt des Garns (Y) während des Entdrillungsvorgangs weniger als ein angemessenes Faservolumen verloren hat und während fes Verdrillungsvorgangs übermäßig stark verdrillt wurde, falls der gegebene Abschnitt des Garns (Y) in einen Bereich (M3) fällt, in dem das Garn bezüglich eines Bereichs (M1) einer angemessenen Dicke und eines angemessenen Faservolumens dünner ist und ein größeres Faservolumen aufweist.

11. Das Verfahren gemäß einem der Ansprüche 1 bis 10, das ferner ein Zuführen des Garns durch eine Garnzufuhrvorrichtung (2, 3), die entlang eines Garnpfades des laufenden Garns (y) in Verarbeitungsrichtung vor der Garnüberwachungsvorrichtung (5) angeordnet ist, und ein Wickeln des Garns durch eine Wickelvorrichtung (7), die entlang des Garnpfades in Verarbeitungsrichtung nach der Garnüberwachungsvorrichtung (5) angeordnet ist, aufweist.

12. Das Verfahren gemäß Anspruch 11, das ferner ein Zusammenführen des Garns (Y), das durch die Garnzusammenführungsvorrichtung (6) geschnitten wurde, die entlang des Garnpfades zwischen der Garnzufuhrvorrichtung (2, 3) und der Garnüberwachungsvorrichtung (5) angeordnet ist, aufweist.

13. Das Verfahren gemäß Anspruch 12, das ferner ein Steuern, durch eine Steuerung (10), der Garnzusammenführungsvorrichtung (6) aufweist, wenn das Garnqualitätsbewertungssegment (57) der Garnüberwachungsvorrichtung (5) die Garnqualität bezüglich einer fehlerhaften Garnzusammenführung bewertet.

14. Das Verfahren gemäß Anspruch 13, bei dem
ein Entdrillungssegment (19a) der Garnzusammenführungsvorrichtung (6) zwei Garnenden des Garns (Y), das geschnitten wurde, entdrillt, und ein Verdrillungssegment (19b) der Garnzusammenführungsvorrichtung die zwei Garnenden, die durch das Entdrillungssegment (19a) entdrillt wurden, verdrillt, und
die Steuerung (10) zumindest entweder das Entdrillungssegment (19a) und/oder das Verdrillungssegment (19b) steuert.

## Revendications

1. Procédé d'évaluation de la qualité d'un fil en défilement à l'aide d'un dispositif de surveillance de fil, le dispositif de surveillance de fil comprenant un premier capteur (51) adapté pour détecter une épaisseur d'un fil en défilement (Y) et pour obtenir une valeur de détection d'épaisseur, un deuxième capteur (52) adapté pour détecter un volume de fibre du fil en défilement (Y) et pour obtenir une valeur de détection de volume de fibre, et un segment d'évaluation de qualité de fil (57), le procédé comprenant les étapes suivantes réalisées par le segment d'évaluation de qualité de fil consistant à:
évaluer la qualité de fil en termes de présence ou d'absence d'un défaut de fil en jugeant une partie comme présentant un défaut de fil si la valeur de détection d'épaisseur et la valeur de détection de volume de fibre de la partie donnée dans le fil en défilement (Y) se situent en dehors d'une plage de défauts de fil admissible (A1) qui est établie à l'aide d'au moins l'épaisseur et le volume de fibre comme paramètres,
s'il est jugé que la partie donnée présente un défaut de fil, amener le fil (Y) à être coupé et amener un moyen de commande de dispositif de jonction de fil (57) à commander un dispositif de jonction de fil (6) pour effectuer une opération de jonction de fil, pour éliminer la partie qui est jugée comme présentant un défaut de fil et pour générer une jonction de fil,
évaluer la qualité du fil en défilement (Y) en termes de présence ou d'absence d'une jonction de fil défectueuse sur base de la valeur de détection d'épaisseur détectée par le premier capteur (51) et de la valeur de détection de volume de fibre détectée par le deuxième capteur (52), en jugeant la jonction de fil comme étant défectueuse si la valeur de détection d'épaisseur et la valeur de détection de volume de fibre de la jonction de fil dans le fil en défilement (Y) se situent en dehors d'une plage de jonction de fil défectueuse admissible (A2) qui est établie à l'aide d'au moins l'épaisseur et le volume de fibre comme paramètres.

2. Procédé selon la revendication 1, dans lequel la qualité de fil se rapporte à une densité de fibre du fil.

3. Procédé selon l'une quelconque des revendications 1 et 2, comprenant par ailleurs le fait de régler, par un segment de réglage (56), au moins l'une parmi la valeur de détection d'épaisseur et la valeur de détection de volume de fibre de sorte que l'épaisseur et le volume de fibre d'une partie donnée dans le fil en défilement (Y) soient corrélés, même si le premier capteur (51) et le deuxième capteur (52) sont situés à des positions différentes le long d'un trajet du fil en défilement (T),
dans lequel la qualité de fil de la partie donnée est évaluée par le segment d'évaluation de qualité de fil (57) sur base de la valeur de détection d'épaisseur et de la valeur de détection de volume de fibre de la partie donnée réglées par le segment de réglage (56).

4. Procédé selon la revendication 3, comprenant par ailleurs le fait de:
acquérir une vitesse de défilement du fil (Y) par un segment d'acquisition de vitesse de fil (55); et
par un segment d'échantillonnage (53, 54), échantillonner la valeur de détection d'épaisseur et la valeur de détection de volume de fibre sur base de la vitesse de défilement acquise par le segment d'acquisition de vitesse de fil (55),
dans lequel au moins l'une parmi la valeur de détection d'épaisseur et la valeur de détection de volume de fibre est réglée par le segment de réglage (56) sur base de la valeur de détection d'épaisseur et de la valeur de détection de volume de fibre échantillonnée par le segment d'échantillonnage (53, 54).

5. Procédé selon l'une quelconque des revendications 1 à 4, dans lequel le premier capteur (51) est un capteur optique.

6. Procédé selon l'une quelconque des revendications 1 à 5, dans lequel le deuxième capteur (52) est un capteur capacitif.

7. Procédé selon l'une des revendications 1 à 6, dans lequel la plage de défauts de fil admissible (A1) et la plage de jonction de fil défectueuse admissible (A2) sont établies respectivement autour du rapport π x (T/2)² entre l'épaisseur T du fil et le volume de fibre en fixant une plage admissible de densité de fibre et en fixant également des limites supérieure et inférieure.

8. Procédé selon l'une des revendications 1 à 7, dans lequel la qualité de fil est évaluée par le segment d'évaluation de qualité de fil (57) en termes de l'état torsadé du fil.

9. Procédé selon la revendication 8, dans lequel il est évalué, par le segment d'évaluation de qualité de fil (57), qu'une partie donnée du fil (Y) a perdu plus d'un volume approprié de fibre pendant une opération de détorsion et a été torsadée de manière inappropriée pendant une opération de torsion si la partie donnée du fil (Y) tombe dans une plage (M2) dans laquelle le fil est plus épais, mais présente un volume de fibre plus petit par rapport à une plage (M1) d'une épaisseur appropriée et d'un volume de fibre approprié.

10. Procédé selon la revendication 8 ou 9, dans lequel il est évalué, par le segment d'évaluation de qualité de fil (57), qu'une partie donnée du fil (Y) a perdu moins d'un volume approprié de fibre pendant l'opération de détorsion et a été torsadée de manière excessive pendant l'opération de torsion si la portion donnée du fil (Y) tombe dans une plage (M3) dans laquelle le fil est plus mince et présente un plus grand volume de fibres par rapport à une plage (M1) d'une épaisseur appropriée et d'un volume de fibre approprié.

11. Procédé selon l'une quelconque des revendications 1 à 10, comprenant par ailleurs le fait d'alimenter le fil par un dispositif d'alimentation de fil (2, 3) qui est disposé en amont du dispositif de surveillance de fil (5) le long d'un trajet du fil en défilement (y), et de bobiner le fil par un dispositif de bobinage (7) qui est disposé en aval du dispositif de surveillance de fil (5) le long du trajet de fil.

12. Procédé selon la revendication 11, comprenant par ailleurs le fait de joindre le fil (Y) qui a été coupé par le dispositif de jonction de fil (6) qui est disposé entre le dispositif d'alimentation de fil (2, 3) et le dispositif de surveillance de fil (5) le long du trajet de fil.

13. Procédé selon la revendication 12, comprenant par ailleurs le fait de commander, par un dispositif de commande (10), le dispositif de jonction de fil (6) lorsque le segment d'évaluation de qualité de fil (57) du dispositif de surveillance de fil (5) évalue la qualité du fil en termes d'une jonction de fil défectueuse.

14. Procédé selon la revendication 13, dans lequel
un segment de détorsion (19a) du dispositif de jonction de fil (6) défait la torsion de deux extrémités du fil (Y) qui a été coupé, et un segment de torsion (19b) du dispositif de jonction de fil torsade les deux extrémités de fil dont la torsion a été défaite par le segment de détorsion (19a), et
le moyen de commande (10) commande au moins l'un parmi le segment de détorsion (19a) et le segment de torsion (19b).
